(19) Europäisches Patentamt
European Patent Office
Office européen des brevets

(11) Publication number: 0 015 615
B1

(12) **EUROPEAN PATENT SPECIFICATION**

(45) Date of publication of patent specification: 12.01.83

(51) Int. Cl.³: C 07 D 295/12, C 07 D 295/22, A 61 K 31/495

(21) Application number: 80200173.5

(22) Date of filing: 28.02.80

(54) **Phenyl piperazine derivatives, their preparation and antiagressive medicines containing them.**

(30) Priority: 01.03.79 NL 7901633

(43) Date of publication of application: 17.09.80 Bulletin 80/19

(45) Publication of the grant of the patent: 12.01.83 Bulletin 83/2

(84) Designated Contracting States: AT BE CH DE FR GB IT LU NL SE

(56) References cited:
GB - A - 1 368 256
GB - A - 1 443 598
GB - A - 1 490 507
US - A - 2 722 529
US - A - 2 833 770

(73) Proprietor: DUPHAR INTERNATIONAL RESEARCH B.V
C.J. van Houtenlaan 36
1381 CP Weesp (NL)

(72) Inventor: Haeck, Hans Heinz
c/o OCTROOIBUREAU ZOAN B.V. Apollolaan 151
NL-1077 AR Amsterdam (NL)
Inventor: Hillen, Feddo Cornelius
c/o OCTROOIBUREAU ZOAN B.V. Apollolaan 151
NL-1077 AR Amsterdam (NL)

(74) Representative: Muis, Maarten, Drs. et al,
OCTROOIBUREAU ZOAN B.V. Apollolaan 151
NL-1077 AR Amsterdam (NL)

EP 0 015 615 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

Phenyl piperazine derivatives, their preparation and antiaqressive medicines containing them

The invention relates to new phenyl piperazine derivatives, to the preparation of these compounds, and to compositions containing at least one of these compounds as an active substance.

Various phenyl piperazine derivatives having a variety of pharmacological properties are known.

For example, United States Patent Specification 2,722,529 relates to compounds of formula 1 of the formula sheet, in which Y is a straight or branched alkyl group having 2—6 carbon atoms and Z is an acyl group, a sulphonyl group or a carbamoyl group. These compounds are said to have a strong sympatholytic activity and sometimes a hypotensive effect.

Furthermore, United States Patent Specification 2,833,770 discloses a group of compounds of the general formula 2 of the formula sheet, in which formula R is a chlorine atom or a bromine atom, R' is a hydrogen atom or a methyl group and n is an integer of 2—5. Of these compounds also a sympatholytic effect is known.

It has now been found that phenyl piperazine derivatives of formula 3 of the formula sheet, in which $R_1$ is a hydrogen atom, a methyl group, an alkoxy group having 1 or 2 carbon atoms, an amino group or a ureido group, $R_2$ is a hydrogen atom or an alkyl group having 1—3 carbon atoms, $R_3$ a halogen atom or trifluoromethyl group, T is an oxygen or sulfur atom, and n is 0 or 1, and the salts thereof, have a strong antiagressive activity.

When $R_2$ represents an alkyl group, this preferably is the methyl group or ethyl group. When $R_3$ is halogen this preferably is fluorine, chlorine or bromine.

On the basis of their activity pattern and chemical accessibility and stability, the following compound is to be preferred:

1-[2-(N-aminocarbonylamino)ethyl]-4-(3-trifluoromethylphenyl)piperazine

It has surprisingly been found that this antiagessive activity of the compounds according to the invention is not associated with the sympatholytic properties described for the above-mentioned known and structurally closely related compounds, which properties are undesired for antiagressiveness.

Other side effects undesired also for use as antiagressives, for example, dopaminolytic, muscle relaxing and sedative properties are absent in the dosages in which the antiagressive effect occurs.

It is surprising that the compounds according to the invention show such a strong and in particular selective antiagressive activity, since structurally very closely related known compounds have quite a different pharmacological activity pattern.

In the table below, the antiagressive activity of a few compounds according to the invention is compared with two compounds known from US—A—2,722,529 and with a related compound which falls within the scope of protection of US—A—2,833,770.

Both the new compounds according to the invention recorded in the table and the known compounds fall under the general formula 4 of the formula sheet, in which the symbols have the meanings indicated in the table:

TABLE

| $R_4$ | $R_5$ | $R_6$ | T | m | p | $ED_{50}$ | Remarks |
|---|---|---|---|---|---|---|---|
| $CH_3$ | H | 3-Cl | O | 0 | 2 | 1.8 | new compound |
| $CH_3$ | H | 3-$CF_3$ | O | 0 | 2 | 0.7 | " " |
| $NH_2$ | H | 3-Cl | O | 0 | 2 | 2.3 | " " |
| $NH_2$ | H | 3-$CF_3$ | O | 0 | 2 | 1.3 | " " |
| H | H | 3-Cl | O | 0 | 2 | 1.5 | " " |
| H | H | 3-$CF_3$ | O | 0 | 2 | 1.7 | " " |
| $CH_3$ | $CH_3$ | 3-$CF_3$ | O | 0 | 2 | 0.9 | " " |
| $CH_3$ | H | 3-Br | O | 0 | 2 | 2.6 | " " |
| $CH_3$ | $C_2H_5$ | 3-$CF_3$ | O | 0 | 2 | 3.2 | " " |
| $CH_3O$ | H | 3-Cl | O | 0 | 2 | 3.5 | " " |
| $CH_3O$ | H | 3-$CF_3$ | O | 0 | 2 | 0.6 | " " |
| $CH_3$ | H | 3-$CF_3$ | S | 0 | 2 | 1.6 | " " |
| $NH_2CONH$ | H | 3-Cl | O | 0 | 2 | 2.3 | " " |
| $NH_2CONH$ | H | 3-$CF_3$ | O | 0 | 2 | 1.5 | " " |
| $CH_3$ | H | 3-$CF_3$ | O | 1 | 2 | 1.5 | " " |
| $NH_2$ | H | 3-$CF_3$ | O | 1 | 2 | 1.9 | " " |
| $CH_3$ | H | H | O | 0 | 2 | 15 | US pat. spec. 2.722.529 ex. 12 |
| $NH_2$ | H | H | O | 0 | 3 | 46 | US pat. spec. 2.722.529 ex. 7 |
| $CH_3$ | H | 2-Cl | O | 0 | 2 | 18 | US pat. spec. 2.833.770 |

The $Ed_{50}$-values mentioned in the table were determined in a test on antiagressive activity in isolated mice (Advances in Pharmacol. *5*, (1967), 79). In this test, male albino mice were kept isolated for a period of 4 weeks and then selected for the test on the basis of fighting behaviour present. The selection criteron is the occurrence of 3 or more fighting periods within 3 minutes after a mouse which has not been kept isolated has been placed in the cage of the mouse kept isolated.

The compounds to be investigated were administered orally to the selected mice. Per dose 5 mice were used. Sixty minutes after the administration of the compound to be investigated the animals were evaluated again for fighting behaviour. The compound to be investigated is inactive in the administered dose when now also 3 or more fighting periods were observed within 3 minutes after the mouse not kept isolated was placed in the cage of the mouse kept isolated. The $ED_{50}$-value in mg of active substance per kg of body weight was calculated from the results obtained.

It appears from the found $ED_{50}$-values that the new compounds according to the invention show a much stronger antiagressive activity than the three known compounds.

Since in the compounds according to the invention the sympatholytic activity and also other undesired side effects mentioned of the known compounds were not found, the new compounds are excellently suitable -due to their surprisingly selective antiagressive activity pattern- for being used in the treatment of agressive behaviour in man and animal.

For use in human medicine are to be considered first of all the control of aggressive symptoms in psychiatric diseases and serious forms of psychopathological agression.

As application possibilities in the veterinary field are to be considered in particular those forms of

agression which occur in transporting agricultural domestic animals and the mixing of groups of these animals.

The quantity, frequency, and way of administration may differ for each individual case, also dependent on the nature and severity of the disturbances. For human application a dosage of from 5—500 mg and preferably from 25—150 mg daily, depending on the way of administration, will generally be suitable.

For veterinary purposes the dosage is preferably 0.1—10 mg/kg of body weight, depending on the way of administration.

The active compounds according to the invention and their salts can be processed according to known standard methods to compositions such a pills, tablets, coated tablets, capsules, powders and injection liquids while using the usual auxiliary substances such as solid and liquid carrier materials.

As examples of pharmaceutically acceptable acids with which the compounds according to the invention can form salts may be mentioned hydrochloric acid, sulphuric acid, nitric acid, citric acid, fumaric acid, maleic acid, tartaric acid, methane sulphonic acid and benzoic acid.

The compounds of formula 3 and their salts can be prepared by methods suitable for the synthesis of analogous compounds. The invention therefore also relates to the preparation of the new compounds and the salts thereof.

Depending on the meanings of the symbols $R_1$—$R_3$,T and m the compounds of general formula 3 can be obtained by at least one of the following methods:

a) Reaction of a compound of formula 5 with an ester (preferably the methyl ester or ethyl ester) or the amide or formic acid or acetic acid.

The reaction with an ester is preferably carried out with an excess of the ester, so that the ester also serves as a solvent, at a temperature between room temperature and the boiling point of the ester used.

The reaction with the amide is preferably carried out without a solvent at elevated temperature (see J. Pharm. Soc. Japan, *62* (1942), 531).

b) Reaction of a compound of formula 5 with an alkali metal cyanate, urea or nitrobiuret.

The reaction with an alkali metal cyanate is preferably carried out in water to which an equivalent amount of an acid, for example, hydrochloric acid or acetic acid, has been added. The reaction mixture is usually stirred at room temperature for a few hours.

The reaction with urea is preferably carried out at a temperature of 130—150°C without a solvent.

c) Reaction of a compound of formula 5 with an acetic acid halide, the anhydride of formic acid and acetic acid, acetic acid anhydride, or with a methoxy- or ethoxycarbonlhalide. This reaction is carried out in an organic solvent, for example acetic acid, methylene chloride or tetrahydrofuran, at temperatures between room temperature and the boiling point of the solvent used.

d) Reaction of a compound of formula 5 with formic acid or acetic acid. The reaction is preferably carried out with an excess of the acid as a solvent. An inert organic solvent may also be used, for example, xylene, whether or not in combination with a water-absorbing agent or "water trap". The reaction temperature generally is between room temperature and the boiling point of the solvent used.

Compounds according to the invention can also be obtained by reaction of a compound of formula 6 with a compound of formula 7. In these formulae, $R_1$, $R_2$ and $R_3$ have the above meanings and X is a so-called leaving group, preferably chlorine, bromine or tosylate. The reaction may be carried out both with and without an inert organic solvent. Suitable solvents are, for example, methyl ethyl ketone, dimethyl formamide, tetrahydrofuran and petroleum ether. As an acid-binding agent is preferably used triethyl amine or potassium carbonate. The reaction temperature is usually between room temperature and 60°C and the reaction duration varies from a few hours to approximately 20 hours.

Compounds according to the invention of formula 3 in which $R_1$ is e.g. methyl, $R_2$ is an alkyl group and $R_3$ has the above-mentioned meaning can also be prepared by reaction of a compound of formula 3 not substituted at the nitrogen atom with a compound $R_2Y$, in which $R_2$ is alkyl and Y is halogen (preferably iodine) or $(SO_4)_{\frac{1}{2}}$.

This reaction is usually carried out in a solvent such as benzene, DMSO or dimethyl formamide in the presence of a strong base, for example, KOH or NaOH under anhydrous conditions at temperatures between room temperature and reflux temperature of the solvent used (see J. Org. Chem. *14* (1949), 1099).

In similar circumstances, compounds of formula 3 in which $R_1$ is methyl can be prepared by reaction of a compound of formula 8 with a compound of formula 9, in which formulae $R_2$, $R_3$ and X have the above meanings and $R_1$ is methyl.

Furthermore, compounds of formula 3 can be prepared by hydrogenation of a compound of formula 10 with hydrogen and a suitable catalyst (for example platinum oxide or Raney nickel in the presence of a base such as sodium acetate or sodium hydroxide) in acetic acid anhydride and, if desired, in the presence of acetic acid. The reaction is preferably carried out at elevated temperature and pressure, for example at 50°C and $3.5 \times 10^5$ Pa. hydrogen pressure (see J. Org. Chem. *25*, (1960), 1658—1660).

Compounds according to the invention of formula 3 in which $R_3$ is trifluoromethyl, can be obtained by reaction of a compound of formula 11 in which $R_3$ is trifluoromethyl and X is halogen,

preferably bromine, with a compound of formula 12, in which $R_1$ and $R_2$ have the above meanings.

This reaction is carried out in a solvent for example DMSO, in the presence of an acid-binding agent, for example $Na_2CO_3$. The reaction mixture is stirred at elevated temperature (100—180°C) for a few hours (see DE—A—2.024.826).

Furthermore, the compounds of formula 3 can be prepared by converting a compound of formula 13 in which $R_1$, $R_2$ and $R_3$ have the above meanings, with 1,2-dibromoethane. The reaction is carried out in an organic solvent, for example, dioxan or butanol, in the presence of an acid-binding agent, for example $K_2CO_3$ at a temperature of 20—120°C (see Arzneimittel Chemie *27* (11), (1977), 2077—2086).

Compounds of formula 3 in which $R_3$ is chlorine or bromine, can also be prepared by diazotization of the corresponding compound in which $R_3$ is a amino group, with $NaNO_2$ and HCl or HBr. This reaction step is carried out in an excess of the diluted acid to which another acid, for example sulphuric acid, may be added so as to increase the reactivity, at a temperature of from −10 to +5°C. The resulting reaction mixture is then treated with cuprous chloride or cuprous bromide or with copper at 20—75°C, preferably with the addition of a quantity of concentrated HCl or HBr, respectively, the 3-chloro or the 3-bromo compound, respectively, being formed.

The compounds of formula 3 can also be obtained by reacting a compound of formula 14 with a compound of formula 15, in which formulae $R_1$, $R_2$ and $R_3$ have the above meanings. This reaction is preferably carried out in a solvent, for example butanol, in the presence of an acid-binding agent, such as $K_2CO_3$, at temperatures between room temperature and the boiling point of the solvent used (see GB—A—943.739).

Furthermore, the compounds of formula 3 can be prepared in similar reaction conditions by reaction of a compound of formula 16 with a compound of formula 17, in which formulae $R_1$ $R_2$ and $R_3$ have the above meansings (see Coll. Czech. Chem. Comm. *6* (1934), 211).

Compounds of the formula 3 of the formula sheet wherein T is a sulphur atom can be obtained by reacting a correspondent compound of the formula 3 wherein T is oxygen with phosphorus pentasulfide in a suitable solvent such as xylene, toluene, pyridine, dioxan, diethyl ether, dichloromethane or acetonitrile. This reaction is preferably carried out in the presence of sodium bicarbonate or triethylamine to solubilize the phosphorus pentoxide, at temperatures between 0°C and room temperature (see Indian J. Chem. (B) *14*, 1976, page 999, and Synthesis *1973*, 149).

Compounds of the formula 3 wherein T represents a sulphur atom can also be obtained by reacting a compound of the formula 5 with a compound of the formula R″NCS, wherein R″ represents a group which can be easily removed, e.g. an optionally substituted aroyl group. Very suitable for this purpose is the benzoyl group. The so obtained substituted thiourea derivative can be hydrolised with a base (e.g. 2N NaOH) in a solvent such as ethanol. The reaction with R″NCS is carried out in a solvent such as for example acetone or acetonitrile at temperatures between room temperature and the boiling point of the solvent used.

Compounds of the formula 3 wherein m is 1 can be prepared by oxidation of the corresponding compound wherein m is 0. Suitable oxidation agents are hydrogen peroxide, perbenzoic acid, monoperphthalic acid, m-chloroperbenzoic acid, peracetic acid and complexes with hydrogen peroxide. This oxidation reaction can be carried out at temperatures between 0°C and the boiling point of the solvent used. Suitable solvents are inert solvents such as water, mixtures of water and acetone and organic solvents such as alcohols, (e.g. ethanol), ketones (e.g. acetone), acids (e.g. acetic acid), esters (e.g. ethylacetate) and chlorinated hydrocarbons such as $CH_2Cl_2$.

The invention will be described in greater detail with reference to the following examples.

### Example I

*1-[2-(N-formylamino)ethyl]-4-(3-chlorophenyl)piperazine*

16.7 mmol (4.0 g) of 1-(2-aminoethyl)-4-(3-chlorophenyl)piperazine were refluxed for 2 hours in 20 ml of formic acid ethyl ester. The reaction was then evaporated to dryness under reduced pressure and after the addition of 10 ml of toluene, it was again evaporated to dryness so as to remove the excess of ester. The residue was crystallized from toluene/petroleum ether. The crystallisate was purified chromatographically over silica gel with $CH_2Cl_2$/methanol (95:5) as an eluent. After evaporating the solvents, the resulting free base was crystallized from ether/petroleum ether, the title compound being obtained with a melting-point of 66.5—68°C.

### Example II

*1-[2-N-formlamino)ethyl]-4-(3-trifluoromethylphenyl)piperazine hydrochloride*

The free base of the title compound was obtained in a identical manner from 1-(2-aminoethyl)-4-(3-trifluoromethylphenyl)piperazine.

This free base was dissolved in absolute ethanol and an equimolar amount of 2N alcoholic hydrochloric acid was added. After the addition of ether the hydrochloride crystallized out. The resulting title compound had a melting-point of 163—165°C.

### Example III

*1-[2-(N-aminocarbonylamino)ethyl]-4-(3-chlorophenyl)piperazine*

25 Mmol (6.0 g) of 1-(2-aminoethyl)-4-(3-chlorophenyl)piperazine were dissolved in 50 ml of water and 25 ml of 2 N hydrochloric acid. A solution of 60 mmol (4.8 g) of potassium cyanate in 37.5 ml of water was added dropwise while stirring at room temperature. After standing overnight at room temperature, $Na_2CO_3$ was added to the suspension until basic reaction of the mixture. The crystallisate was sucked off and crystallized from 40 ml of ethanol. The title compound obtained in this manner had a melting-point of 163—164°C.

3.5 Mmol (1.0 g) of the title compound were dissolved in 16 ml of absolute ethanol while heating. An equimolar quantity of 2 N alcoholic hydrochloric acid was added. After cooling to room temperature, ether was added until crystallization. The 1-[2-(N-aminocarbonylamino)ethyl]-4-(3-chlorophenyl)-piperazine hydrochloride obtained in this manner had a melting-point of 186—187°C.

3.5 Mmol (1.0 g) of the title compound were dissolved in 13 ml of absolute ethanol while heating. An equimolar quantity of methane sulphonic acid dissolved in 1 ml of absolute ethanol was added. After cooling, 5 ml of ether were added and after standing overnight at room temperature the crystallisate was sucked off, washed and dried. The 1-[2-(n-aminocarbonylamino)ethyl]-4-(3-chlorophenyl piperazine methane sulphonate obtained in this manner had a melting-point of 166.5—167°C.

3.4 Mmol (0.96 g) of the title compound were dissolved in 15 ml of absolute ethanol while heating. 1.7 Mmol of $H_2SO_4$ (3.22 ml of a 1.059 N $H_2SO_4$ solution in water) were added. The solvents were distilled off under reduced pressure at a bath temperature of 50°C. The residue was crystallized from ethanol/ether. The 1-[2-(N-aminocarbonylamino)ethyl]-4-(3-chlorophenyl) piperazine sulphate (2:1) obtained in this manner had a melting-point of 176—177°C.

### Example IV

*1-[2-(N-aminocarbonylamino)ethyl]-4-(3-trifluoromethyphenyl) piperazine*

In the manner described in example III, the title compound (melting point 143—145°C) was obtained from 1-(2-aminoethyl)-4-(3-trifluoromethylphenyl) piperazine.

3.1 Mmol (1.0 g) of the title compound were dissolved in 3 ml of absolute ethanol while heating. An equimolar quantity of 2 N alcoholic hydrochloric acid was added. After cooling to room temperature, ether was added until crystallisation. The 1-[2-(N-aminocarbonylamino)ethyl]-4-(3-trifluoromethylphenyl) piperazine hydrochloride obtained in this manner had a melting-point of 202—203°C.

3.1 Mmol (1.0 g) of the title compound were dissolved in absolute ethanol while heating. An equimolar quantity of sulphuric acid was added. After cooling, 5 ml of ether were added, after which the salt crystallized. The 1-[2-(N-aminocarbonylamino)ethyl]-4-(3-trifluoromethylphenyl) piperazine hemisulphate (1:1) obtained in this manner has a melting-point of 150—151°C.

In an identical manner, a quantity of the title compound was converted, with methane-sulphonic acid, into 1-[2-(N-aminocarbonylamino)ethyl]-4-(3-trifluoromethylphenyl) piperazine mesylate with a melting-point of 183.5—184°C.

### Example V

*1-[2-(N-aminocarbonyl-N-ethyl)aminoethyl]-4-(3-trifluoromethylphenyl)piperazine*

In the manner described in Example III the title compound (melting point 136—137°C) was obtained from 1-[2-(N-ethylamino)ethyl]-4-(3-trifluoromethylphenyl)piperazine.

### Example VI

*1-[2-(N-acetylamino)ethyl]-4-(3-trifluromethylphenyl)piperazine*

20 Mmol (5.2 g) of 1-(2-aminoethyl)-4-(3-trifluoromethylphenyl)piperazine were dissolved in 30 ml of glacial acetic acid. 37.5 Mmol (3.8 g) of acetic acid anhydride were added to the mixture and the mixture was then refluxed for 2 hours. The mixture was then evaporated to dryness under reduced pressure. A saturated solution of sodium carbonate in water was added to the residue and then 2 extractions were carried out with 100 ml of methylene chloride. After drying over $K_2CO_3$ and distilling off methylene chloride, the residue was crystallized from toluene/petroleum ether. The title compound obtained in this manner had a melting-point of 85—87°C.

In the manner described in Example IV, the title compound was converted -with methane sulphonic acid- into 1-[2-(N-acetylamino)ethyl]-4-(3-trifluoromethylphenyl)piperazine mesylate with a melting-point of 171.5—173.5°C.

3.2 Mmol (1.0 g) of the title compound were dissolved in 5 ml of absolute ethanol while heating. 3.2. Mmol (370 mg) of maleic acid were added. 15 Ml of ether were then added and, upon cooling, the salt crystallized. The 1-[2-(N-acetylamino)ethyl]-4-(3-trifluoromethylphenyl)piperazine maleate (1:1) obtained in this manner had a melting-point of 113—115°C.

In the manner described in Example IV, the title compound was converted -with hydrochloric acid- into 1-[2-(N-acetylamino)ethyl]-4-(3-trifluoromethylphenyl)piperazine hydrochloride with a melting point of 197—199°C.

Example VII

*1-[2-(N-acetyl-N-ethylamino)ethyl]-4-(3-trifluoromethylphenyl)piperazine*

15 Mmol (4.5 g) of 1-[2-(N-ethylamino)ethyl]-4-(3-trifluoromethylphenyl)piperazine were dissolved in 30 ml of methylene chloride. A solution of 15 mmol (1.2 g) of acetyl chloride in 10 ml of methylene chloride was added dropwise, the temperature of the reaction mixture not exceeding 40°C. Stirring at room temperature for 30 minutes was then carried out. The mixture was then evaporated to dryness under reduced pressure.

The residue was crystallized from isopropanol, the title compound being obtained with a melting-point of 179—181°C.

Example VIII

*1-[2-(N-formylamino)ethyl]-4-(3-chlorophenyl)piperazine*

62 Mmol (6.3 g) of acetic acid anhydride and 67 mmol (3.0 g) of formic acid were heated at 50—60°C for 2 hours. A solution of 21 mmol of 1-(2-aminoethyl)-4-(3-chlorophenyl)piperazine in 15 ml of absolute tetrahydrofuran was then added dropwise at such a rate that the temperature of the mixture did not exceed 45°C. After stirring overnight at room temperature, the solvents were removed at 40°C. 16 Ml of water and a quantity of sodium bicarbonate (until neutral reaction) were added to the residue. Two extractions with methylene chloride were then carried out. After washing with water and drying over potassium carbonate, the solvent was removed under reduced pressure. The residue was purified chromatographically over silica gel with ethanol-25% ammonio (95:5) as an eluent. The solvents were removed under reduced pressure and the residue was crystallized from ether/petroleum ether. In this manner the title compound was obtained with a melting-point of 66.5—68°C.

Example IX

*1-[2-(N-formylamino)ethyl]-4-(3-chlorophenyl)piperazine*

16.7 Mmol (4.0 g) of 1-(2-aminoethyl)-4-(3-chlorophenyl)piperazine were dissolved in 20 ml of formic acid. The mixture was stirred overnight at room temperature. 50 Ml of water were then added, succeeded by 50 ml of methylene chloride. The layers were separated and the water layer was extracted again with methylene chloride. The collected organic layers were washed with water and dried. The solvent was removed under reduced pressure. The residue was purified chromatographically over silica gel with methylene chloride/methanol (95:5) as an eluent. After removing the solvent, the residue was crystallized from ether/petroleum ether. The title compound obtained in this manner had a melting-point of 66.5—68°C.

Example X

*1-[2-(N-acetylamino)ethyl]-4-(3-chlorophenyl)piperazine*

45 Mmol (8.7 g) of 1-(3-chlorophenyl)piperazine, 45 mmol (5.4 g) of N-acetyl-2-chloroethyl-amine and 45 mmol (6.2 ml) of triethylamine were combined while stirring and heated at 60—70°C for 20 hours. 50 Ml of 2 N KOH-solution and 100 ml of methylene chloride were then added.The layers were separated and the water layer was extracted with methylene chloride. The collected methylene chloride layers were washed with 15 ml of water and dried over $K_2CO_3$. The solvent was then removed under reduced pressure. The residue was purified chromatographically over silica gel with $CH_2Cl_2$/methanol (95:5) as an eluent. After evaporating the solvents, the resulting free base was crystallized from ethyl acetate/petroleum ether, the title compound being obtained with a melting-point of 96.5—97.5°C.

In the manner described in Example IV, a quantity of the title compound was converted -with hydrochloric acid- into 1-[2-(N-acetylamino)ethyl]-4-(3-chlorophenyl)piperazine hydrochloride with a melting-point of 173—175°C.

In the manner described in Example IV, a quantity of the title compound was converted -with methane sulphonic acid- into 1-[2-(N-acetylamino)ethyl]-4-(3-chlorophenyl)piperazine mesylate with a melting point of 171—173°C.

In the manner described in Example IV, a quantity of the title compound was converted -with sulphuric acid- into 1-[2-(N-acetylamino)ethyl]-4-(3-chlorophenyl)piperazine sulphate (1:1) with a melting point of 121.5—124°C.

Example XI

*1-[2-(N-acetylamino)ethyl]-4-(3-trifluoromethylphenyl)piperazine*

In the same manner as described in Example X, the title compound (melting point 85—87°C) was obtained from 1-(3-trifluoromethylphenyl)piperazine. A quantity of the title compound thus obtained was converted in the manner described in Example IV into 1-[2-(N-acetylamino)ethyl]-4-(3-trifluoromethylphenyl)piperazine sulphate (1:1) with a melting point of 119.5—121.5°C.

Example XII

*1-[2-(N-acetyl-N-methylamino)ethyl]-4-(3-trifluoromethylphenyl)piperazine hydrochloride (1:1)*

In the manner described in Example X, the free base of the title compound was obtained as a resin

from 1-(3-trifluoromethylphenyl)piperazine and N-acetyl-N-methyl-2-chloroethylamine.

In the manner described in Example IV, a quantity of the free base was converted -with hydrochloric acid- into the title compound with a melting point of 182—184°C.

In the manner described in Example IV, a quantity of the free base was converted -with methane sulphonic acid- into 1-[2-(N-acetyl-N-methylamino)ethyl]-4-(3-trifluoromethylphenyl)piperazine mesylate (1:1) with a melting point of 123—124°C.

In the manner described in Example IV, a quantity of the free base was converted -with maleic acid- into 1-[2-(N-acetyl-N-methylamino)ethyl]-4-(3-trifluoromethylphenyl)piperazine maleate (1:1) with a melting point of 123—124°C.

### Example XIII

*1-[2-(N-acetylamino)ethyl]-4-(3-fluorophenyl)piperazine*

In the manner described in Example X, the title compound (melting point 97.5—99°C) was obtained from 1-(3-fluorophenyl)piperazine.

### Example XIV

*1-[2-(N-acetylamino)ethyl]-4-(3-bromophenyl)piperazine*

In the manner described in Example X, the title compound (melting point 97—98.5°C) was obtained from 1-(3-bromophenyl)piperazine.

### Example XV

*1-[2-(N-acetyl-N-methylamino)ethyl]-4-(3-chlorophenyl)piperazine dihydrochloride.$H_2O$*

In the manner described in Example X, the title compound (melting point 186—191°C) was obtained from 1-(3-chlorophenyl)piperazine and N-acetyl-N-methyl-2-chloroethylamine.

### Example XVI

*1-[2-(N-aminocarbonylamino)ethyl]-4-(3-chlorophenyl)piperazine*

20 Mmol (4.8 g) of 1-(2-aminoethyl)-4-(3-chlorophenyl)piperazine were mixed with 20 mmol (1.2 g) of urea. The mixture was heated at 140°C for 1 hour, ammonia gas escaping. The mixture was then crystallized from ethanol, the title compound being obtained with a melting-point of 163—164°C.

### Example XVII

*1-[2-(N-acetyl-N-methylamino)ethyl]-4-(3-trifluoromethylphenyl) piperazine hydrochloride (1:1)*

5 Mmol of sodium hydride (0.24 g of a 55% suspension in oil) were dissolved in 30 ml of dimethylformamide. 5 Mmol (1.6 g) of 1-[2-(N-acetylamino)ethyl]-4-(3-trifluoromethylphenyl)-piperazine were added. The mixture was stirred at 70°C for 1 hour. After cooling, 6.6 mmol (0.92 g) of methyl iodide were added and stirring was carried out at 40°C for 2 hours. The mixture was then cooled to room temperature and diluted with 100 ml of water. Two extractions with 150 ml of methylene chloride were carried out. The methylene chloride extract was washed twice with 25 ml of water, dried over $K_2CO_3$ and the solvent was evaporated under reduced pressure. The residue was purified chromatographically over silica gel with $CH_2Cl_2$/methanol (7:3) as an eluent. After evaporating the solvents, the resulting free base of the title compound was obtained as a resin. A quantity of the free base was converted in the usual manner with hydrochloric acid into the title compound (melting point 182—184°C).

### Example XVIII

*1-[2-(N-acetylamino)ethyl]-4-(3-trifluoromethylphenyl)piperazine*

40 Mmol (2.8 g) of acetamide were added to a suspension of 40 mmol of NaH (1.9 g of a 55% suspension in oil) in 100 ml of dimethylformamide. The mixture was stirred for 1 hour and heated at 60°C. The mixture was then cooled to room temperature and a solution of 46 mmol (13.6 g) of 1-(2-chloroethyl)-4-(3-trifluoromethylphenyl)piperazine in 15 ml of dimethylformamide was added.

This mixture was then stirred for 2 hours and heated at 40°C. The mixture was then cooled to room temperature and the solvent was removed under reduced pressure. Water and methylene chloride were added to the residue. After separation, the organic extract was dried over $K_2CO_3$ and the solvent was evaporated under reduced pressure. The residue was purified chromatographically over silica gel with $CH_2Cl_2$/methanol (95:5) as an eluent. After evaporating the solvents, the resulting free base was crystallized from ethyl acetate/petroleum ether, the title compound being obtained with a melting-point of 85—87°C.

### Example XIX

*1-[2-(N-acetylamino)ethyl]-4-(3-chlorophenyl)piperazine*

3.8 Mmol (1.0 g) of 1-[2-(N-acetylamino)ethyl]-4-(3-aminophenyl)piperazine were dissolved in 5 ml of 6 N HCl. The mixture was then cooled to 0°C and was diazotized at this temperature with a solution of 4 mmol (0.28 g) of $NaNO_2$ in 2 ml of water. After leaving to stand at 0°C for 20 minutes, the solution was added to a suspension of 4 mmol (0.4 g) of cuprous chloride and a catalytic quantity of

copper powder in 5 ml of water of 70°C. The whole was cooled to room temperature and 5 ml of concentrated HCl were added. The suspension was then stirred for 2 hours and after cooling to 0°C, 5 ml of 50% sodium hydroxide solution were added. Three extractions with methylene chloride were then carried out and the extract was washed with water.The solvent was distilled off under reduced pressure. The residue was purified chromatographically over silica gel with $CH_2Cl_2$/methanol (95:5) as an eluent. After evaporation the solvents, the resulting free base was crystallized with ethyl acetate/petroleum ether, the title compound being obtained with a melting point of 96.5—97.5°C.

Example XX

*1-[2-(N-acetylamino)ethyl]-4-(3-trifluoromethylphenyl)piperazine*

100 mmol (25.7 g) of 1-(cyanomethyl)-4-(3-trifluromethylphenyl)piperazine were dissoved in 120 ml of acetic acid anhydride. Three grams of Raney nickel which was previously washed with absolute ethanol were added to this solution, succeeded by acetic acid anhydride. Hydration with $H_2$-gas was then carried out at room temperature while shaking at 4 atm. After 1 hour the calculated quantity of $H_2$-gas had been taken up and the reaction was discontinued.

The catalyst was removed by filtration and acetic acid anhydride was evaporated under reduced pressure. The residue was purified chromatographically over silica gel with methylene chloride/methanol (95:5) as an eluent. After removing the solvent, the residue was crystallized from toluene/petroleum ether. The title compound obtained in this manner has a melting-point of 85—87°C.

Example XXI

*1-[2-(N-methoxycarbonylamino)ethyl]-4-(3-chlorophenyl)piperazine dihydrochloride*

In 10 minutes 20 mmol (1.8 g) of methylchloroformate were added to a solution of 15 mmol (3,6 g) of 1-(2-aminoethyl)-4-(3-chlorophenyl)piperazine in 40 ml of $CH_2Cl_2$. After stirring for 1 hour the mixture was washed twice with 25 ml of a 5% $NaHCO_3$ solution. The organic layer was dried over $K_2CO_3$ and concentrated to dryness. The so obtained free base was treated with 2 equivalents of HCl in dry ethanol. After addition of 100 ml of ether the title compound precipitated and was filtered by suction. After drying the compound had a melting point of 146.5—148°C.

Example XXII

*1-[2-(N-methoxycarbonylamino)ethyl]-4-(3-trifluoromethylphenyl)piperazine hydrochloride*

A solution of 0.12 mmol (11.3 g) of methylchloroformate in 60 ml of $CH_2Cl_2$ was added with stirring to a solution of 0.1 mmol (27.3 g) of 1-(2-aminoethyl)-4-(trifluoromethylphenyl)piperazine in 400 ml of $CH_2Cl_2$ in 45 minutes. The mixture was stirred for 1 additional hour at room temperature. After standing overnight the precipitated material was collected by suction. After crystallization from 100 ml of dry ethanol the title compound was obtained having a melting point 153—155°C.

Example XXIII

*1-[2-(N-thioacetylamino)ethyl]-4-(3-trifluoromethylphenyl)piperazine hydrochloride*

To a suspension of 10 mmol (3.15 g) of 1-[2-(N-acetylamino)ethyl]-4-(3-trifluoromethylphenyl)-piperazine and 10 mmol (2,22 g) of $P_2S_5$ in 30 ml of $CH_2Cl_2$ 40 mmol (5.12 ml) of triethylamine were added dropwise while stirring at room temperature. After 24 hours the mixture was washed with water and then the organic layer was separated and dried with $K_2CO_3$, the residue obtained after evaporating the solvent was purified by chromatography on silica gel with ethylacetate as eluent. After evaporating the solvent, the so obtained free base was treated with an equivalent amount of HCl in ethanol. The so obtained hydrochloride was crystallized from the mixture by addition of ether and melted at 140—142°C.

Example XXIV

*1-[2-(N-allophanoylamino)ethyl]-4-(3-trifluoromethylphenyl)piperazine*

A solution of 33.7 mmol (9.22 g) of 1-(2-aminoethyl)-4-(3-trifluoromethylphenyl)piperazine and 33.7 mmol (5 g) of nitrobiuret in 125 ml of $H_2O$ was slowly warmed until reflux temperature was reached. After 1 hour at this temperature and 48 hours at room temperature the precipitated material was filtered by suction, dried and crystallized from 30 ml of acetonitrile and 25 ml of ethylacetate respectively. The title compound obtained in this way melted at 149—149,5°C.

Example XXV

*1-[2-(N-allophanoylamino)ethyl]-4-(3-chloromethylphenyl)piperazine*

The title compound was obtained in an identical manner, using 1-(2-aminoethyl)-4-(3-chlorophenyl)piperazine as the starting compound. The so obtained title compound had a melting point of 163—164°C.

Example XXVI

*1-[2-(N-acetylamino)ethyl]-4-(3-trifluoromethylphenyl)piperazine 1-oxide*

To a solution of 9.59 mmol (3.02 g) of 1-[2-(N-acetylamino)ethyl]-4-(3-trifluoromethylphenyl)-piperazine in 40 ml of $CH_2Cl_2$ a solution of 9.6 mmol of 3-chloroperbenzoic acid in 15 ml of $CH_2Cl_2$ was added dropwise. Two hours after the addition the mixture was washed twice with 10 ml of 2 N NaOH and 10 ml of $H_2O$ respectively. The organic layer was dried over $K_2CO_3$ and after evaporation of the solvent and crystallisation of the residue the pure title compound was obtained (melting point 167.5—169°C).

Example XXVII

*1-[2-(N-aminocarbonylamino)ethyl]-4-(3-trifluoromethylphenyl)piperazine 1-oxide*

In an identical manner 1-[2-(N-aminocarbonylamino)ethyl]-4-(3-trifluoromethylphenyl)-piperazine was converted into the corresponding 1-oxide. The so obtained title compound had a melting point of 192.5—193.5°C.

Example XXVIII

*Tablet:*

*50 mg of 1-[2-(N-acetylamino)ethyl]-4-(3-chlorophenyl)piperazine-HCl*
335 mg of lactose
60 mg of potato starch
25 mg of talcum
5 mg of magnesium stearate
5 mg of gelatin

Example XXIX

*Suppository:*

*50 mg of 1-[2-(N-acetylamino)ethyl]-4-(3-chlorophenyl)piperazine.HCl*
1500 mg of suppository mass

Example XXX

*Injection liquid:*

*25 g of 1-[2-(N-acetylamino)ethyl]-4-(3-chlorophenyl)piperazine.HCl*
1.80 g of methyl p-hydroxybenzoate
0.20 g of propyl p-hydroxybenzoate
9.0 g of sodium chloride
26 g of glycerin
1 g of benzyl alcohol
water up to 1.000 g.

**Claims for the Contracting States: BE CH DE FR GB IT LU NL SE**

1. Compounds of formula 3

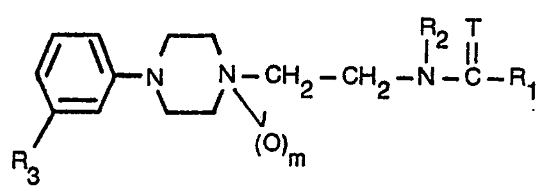

and salts thereof with pharmaceutically acceptable acids, in which formula
$R_1$ is hydrogen, methyl, methoxy, ethoxy, amino or ureido
$R_2$ is hydrogen or an alkyl group having 1—3 carbon atoms,
$R_3$ is trifluoromethyl or halogen,
T is oxygen or sulphur, and
m is 0 or 1.

2. 1-[2-(N-aminocarbonylamino)ethyl]-4-(3-trifluoromethylphenyl)piperazine and salts thereof with pharmaceutically acceptable acids.

3. Pharmaceutical compositions, characterized in that as an active substance they contain at least one compound of formula 3, in which $R_1$, $R_2$, $R_3$, T and m have the meanings mentioned in claim 1, or a pharmaceutically acceptable acid addition salt thereof.

4. A method of preparing pharmaceutically active phenyl piperazine derivatives, characterized in that antiagressively active compounds of formula 3, in which $R_1$, $R_2$, $R_3$, T and m have the meanings mentioned in claim 1, and salts thereof with pharmaceutically acceptable acids are prepared in a manner known for the synthesis of analogous compounds.

5. A method as claimed in claim 4 characterized in that a compound of formula 5

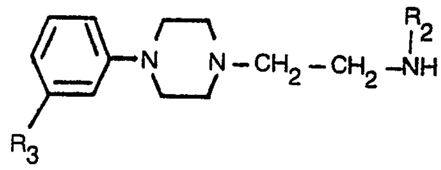

is reacted with

a) an ester or amide of formic acid or acetic acid, or

b) an alkali metal cyanate, urea or nitrobiuret, or

c) an acetic acid halide, acetic acid anhydride, the anhydride of formic acid and acetic acid, or with a methoxy- or ethoxy-halide, or

d) formic acid or acetic acid.

6. A method as claimed in claim 5, characterized in that a compound of formula 3, in which T is sulphur is prepared by converting the corresponding compound wherein T is oxygen, with phosphorous pentasulfide.

7. A method as claimed in claim 5, characterized in that a compound of the formula 3, in which T is sulphur is prepared by reacting a compound of the formula 5 with a compound of the formula R''NCS, wherein R'' represents a group which can easily be removed.

8. A method as claimed in claim 5, characterized in that a compound of the formula 3, in which m is 1 is prepared by oxidation of the corresponding compound wherein m is 0.

**Claims for the Contracting State: AT**

1. A method of preparing pharmaceutically active phenyl piperazine derivatives, characterized in that antiagressively active compounds of formula 3

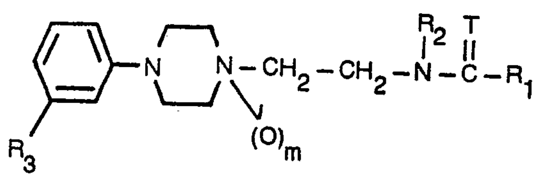

and salts thereof with pharmaceutically acceptable acids, in which formula

$R_1$ is hydrogen, methyl, methoxy, ethoxy, amino or ureido

$R_2$ is hydrogen or an alkyl group having 1—3 carbon atoms,

$R_3$ is trifluoromethyl or halogen,

T is oxygen or sulphur, and

m is 0 or 1.

are prepared according to a method known for the synthesis of analogous compounds.

2. A method as claimed in claim 1, characterized in that a compound of formula 5

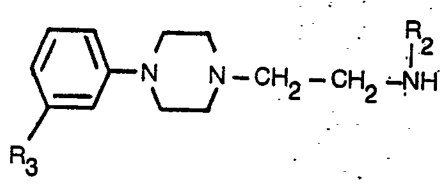

is reacted with

a) an ester or amide of formic acid or acetic acid, or

b) an alkali metal cyanate, urea or nitrobiuret, or

c) an acetic acid halide, acetic acid anhydride, the anhydride of formic acid and acetic acid, or with a methoxy- or ethoxy-halide, or

d) formic acid or acetic acid.

3. A method as claimed in claim 1, characterized in that a compound of formula 6

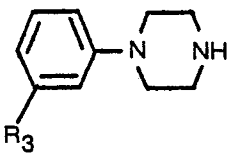

in which $R_3$ have the meaning stated in claim 1, is converted with a compound of formula 7

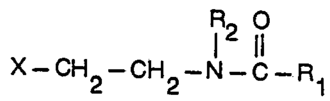

in which $R_1$ and $R_2$ have the meanings stated in claim 1 and X is a leaving group, such as chlorine, bromine or tosylate.

4. A method as claimed in claim 1, characterized in that a compound of formula 3 is prepared in which

$R_1$ is methyl,

$R_2$ is alkyl having 1—3 carbon atoms, and

$R_3$ has the meaning stated in claim 1,

by converting a compound of formula 3 not substituted at the nitrogen atom, with a compound $R_2Y$, in which $R_2$ has the above-mentioned meaning and Y is a halogen atom or sulphate group.

5. A method as claimed in claim 1, characterized in that a compound of formula 8

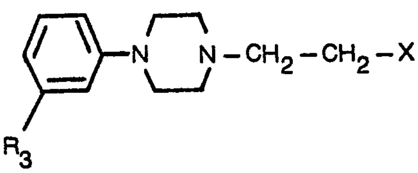

in which $R_3$ has the meaning stated in claim 1 and X has the meaning stated in claim 3, is converted with a compound of formula 9

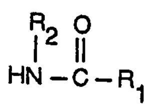

in which $R_2$ has the meaning stated in claim 1 and $R_1$ is methyl.

6. A method as claimed in claim 1, characterized in that a compound of formula 10

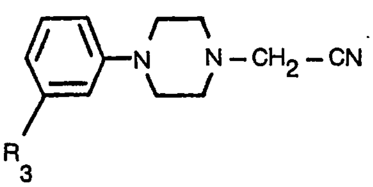

is hydrogenated with hydrogen in the presence of a catalyst in acetic acid anhydride, if desired in the presence of acetic acid.

7. A method as claimed in claim 1, characterized in that a compound of formula 11

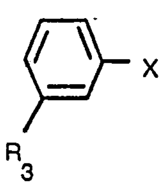

in which X has the meaning stated in claim 3 and $R_3$ is trifluoromethyl, is converted with a compound of formula 12

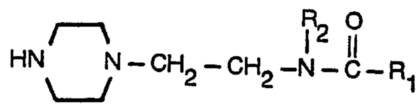

in which $R_1$ and $R_2$ have the meanings stated in claim 1.

8. A method as claimed in claim 1, characterized in that a compound of formula 13

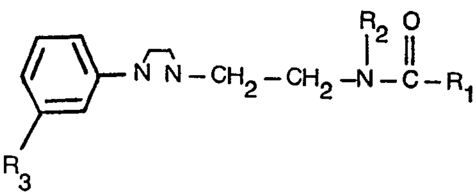

in which $R_1$, $R_2$ and $R_3$ have the meanings stated in claim 1, is converted with 1,2-dibromoethane.

9. A method as claimed in claim 1, characterized in that a compound of formula 3, in which $R_1$ and $R_2$ have the meanings stated in claim 1 and $R_3$ is a halogen atom, is prepared by diazotization of a compound of formula 3, in which $R_1$ and $R_2$ have the meanings stated in claim 1 and $R_3$ is the amino group, with $NaNO_2$ and HCl or HBr, and treating the reaction mixture with cuprous chloride, cuprous bromide or copper.

10. A method as claimed in claim 1, characterized in that a compound of formula 14

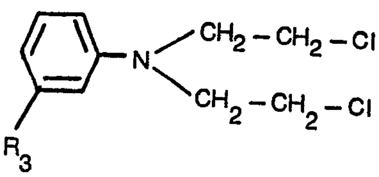

in which $R_3$ has the meaning stated in claim 1, is converted with a compound of formula 15

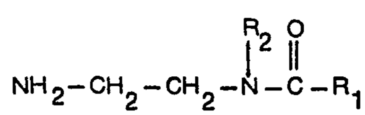

in which $R_1$ have the meanings stated in claim 1.

11. A method as claimed in claim 1, characterized in that a compound of the formula 16

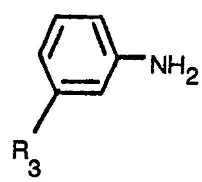

in which $R_3$ has the meaning stated in claim 1, is converted with a compound of formula 17

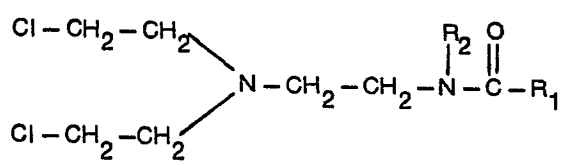

in which $R_1$ and $R_2$ have the meanings stated in claim 1.

12. A method as claimed in claim 1, characterized in that a compound of formula 3, in which T is sulphur is prepared by converting the corresponding compound wherein T is oxygen, with phosphorous pentasulfide.

13. A method as claimed in claim 1, characterized in that a compound of the formula 3, in which T is sulphur is prepared by reacting a compound of the formula 5 with a compound of the formula

R''NCS, wherein R'' represents a group which can easily be removed.

14. A method as claimed in claim 1, characterized in that a compound of the formula 3, in which m is 1 is prepared by oxidation of the corresponding compound wherein m is 0.

**Revendications pour les Etats contractants: BE CH DE FR GB IT LU NL SE**

1. Composés de formule 3

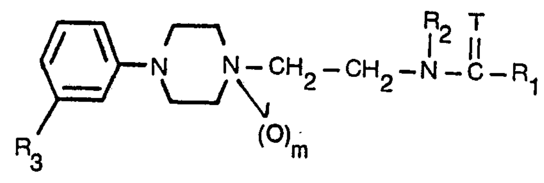

et leurs sels avec des acides pharmaceutiquement acceptables, formule dans laquelle:

$R_1$ représente l'hydrogène ou un groupe méthyle, méthoxy, éthoxy, amino ou uréido,

$R_2$ représente l'hydrogène ou un groupe alkyle ayant de 1 à 3 atomes de carbone,

$R_3$ représente un groupe trifluorométhyle ou un halogène,

T représente l'oxygène ou le soufre, et

m est 0 ou 1.

2. 1-[2-(N-aminocarbonylamino) éthyl]-4-(3-trifluorométhylphényl)pipérazine et ses sels avec des acides pharmaceutiquement acceptables.

3. Compositions pharmaceutiques, caractérisées en ce que, comme substance active, elles contiennent au moins un composé de formule 3, dans laquelle $R_1$, $R_2$, $R_3$, T et m ont les significations mentionnées dans la revendication 1, ou un de leurs sels d'addition avec des acides pharmaceutiquement acceptables.

4. Procédé de préparation de dérivés de phényl pipérazine pharmaceutiquement actifs, caractérisé en ce que des composés de formule 3 à activité antiagressive, dans lesquels $R_1$, $R_2$, $R_3$, T et m ont les significations mentionnées dans la revendication 1, et leurs sels avec des acides pharmaceutiquement acceptables sont préparés d'une manière connue pour la synthèse de composés analogues.

5. Procédé selon la revendication 4, caractérisé en ce qu'on fait réagir un composé de formule 5

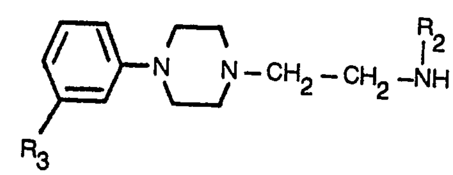

avec

a) un ester ou un amide de l'acide formique ou de l'acide acétique, ou

b) un cyanate de métal alcalin, l'urée ou le nitrobiuret, ou

c) un halogénure de l'acide acétique, l'anhydride de l'acide acétique, l'anhydride de l'acide formique et l'acide acétique, ou avec un méthoxy- ou un éthoxy-halogénure, ou

d) l'acide formique ou l'acide acétique.

6. Procédé selon la revendication 4, caractérisé en ce qu'on prépare un composé de formule 3 où T est le soufre, par conversion du composé correspondant dans lequel T est l'oxygène, avec le pentasulfure phosphoreux.

7. Procédé selon la revendication 4, caractérisé en ce qu'on prépare un composé de formule 3 où T est le soufre, par réaction d'un composé de formule 5 avec un composé de formule R''NCS, dans laquelle R'' représente un groupe facilement éliminable.

8. Procédé selon la revendication 4, caractérisé en ce qu'on prépare un composé de formule 3, dans lequel m est 1, par oxydation du composé correspondant dans lequel m est 0.

**Revendications pour l'Etat contractant: AT**

1. Procédé de préparation de derivés de phényl pipérazine pharmaceutiquement actifs, caractérisé en ce que des composés possédant une activité anti-agressive de formule 3

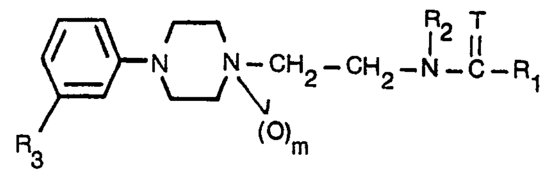

formule dans laquelle:

$R_1$ représente l'hydrogène ou un groupe méthyle, méthoxy, éthoxy, amino ou uréido,

$R_2$ représente l'hydrogène ou un groupe alkyle ayant de 1 à 3 atomes de carbone,

$R_3$ représente un groupe trifluorométhyle ou un halogène,

T représente l'oxygène ou le soufre, et

m est 0 ou 1,

sont préparés selon un procédé connu pour la synthèse de composés analogues.

2. Procédé selon la revendication 1, caractérisé en ce qu'on fait réagir un composé de formule 5

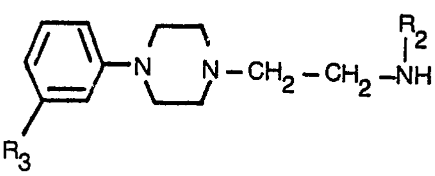

avec

a) un ester ou un amide de l'acide formique ou de l'acide acétique, ou

b) un cyanate de métal alcalin, l'urée ou le nitrobiuret, ou

c) un halogénure de l'acide acétique, l'anhydride de l'acide acétique, l'anhydride de l'acide formique et l'acide acétique, ou avec un méthoxy- ou éthoxy-halogénure, ou

d) l'acide formique ou l'acide acétique.

3. Procédé selon la revendication 1, caractérisé en ce qu'on convertit un composé de formule 6

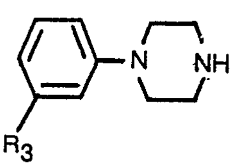

dans laquelle $R_3$ a la signification définie dans la revendication 1, avec un composé de formule 7

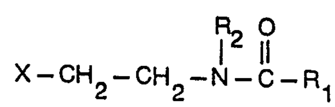

dans laquelle $R_1$ et $R_2$ ont les significations indiquées dans la revendication 1 et X est un groupe éliminable tel que chlore, brome ou tosylate.

4. Procédé selon la revendication 1, caractérisé en ce qu'on prépare un composé de formule 3, où

$R_1$ est un groupe méthyle,

$R_2$ est un groupe alkyle ayant de 1 à 3 atomes de carbone, et

$R_3$ a la signification définie dans la revendication 1, en convertissant un composé de formule 3, non substitué sur l'atome d'azote, avec un composé $R_2Y$ dans laquel $R_2$ a la signification indiquée ci-dessus et Y est un atome d'haogène ou un groupe sulfate.

5. Procédé selon la revendication 1, caractérisé en ce qu'on convertit un composé de formule 8

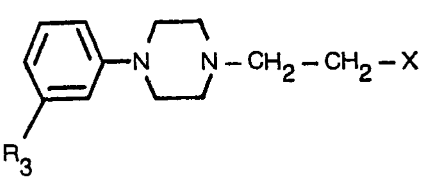

dans laquelle $R_3$ est tel que défini dans la revendication 1 et X a la signification indiquée dans la revendication 3, avec un composé de formule 9

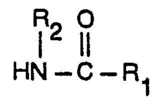

dans laquelle $R_2$ a la signification indiquée dans la revendication 1 et $R_1$ est un groupe méthyle.

6. Procédé selon la revendication 1, caractérisé en ce qu'on hydrogène un composé de formule 10

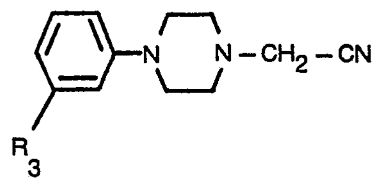

avec de l'hydrogène en présence d'un catalyseur dans de l'anhydride acétique, et si on le souhaite en présence d'acide acétique.

7. Procédé selon la revendication 1, caractérisé en ce qu'on convertit un composé de formule 11

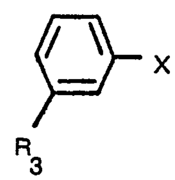

dans laquelle X a la signification indiquée dans la revendication 3 et $R_3$ est ùn groupe trifluorométhyle, avec un composé de formule 12

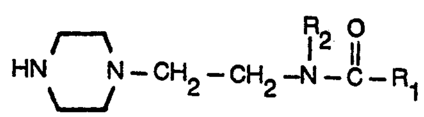

dans laquelle $R_1$ et $R_2$ ont les significations indiquées dans la revendication 1.

8. Procédé selon la revendication 1, caractérisé en ce qu'on convertit un composé de formule 13

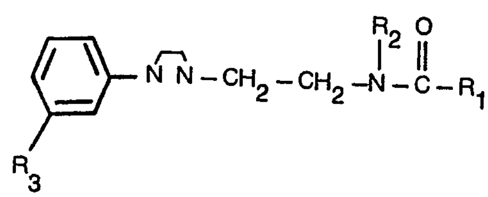

dans laquelle $R_1$, $R_2$ et $R_3$ ont les significations indiquées dans la revendication 1 avec le 1,2-dibromoéthane.

9. Procédé selon la revendication 1, caractérisé en ce qu'on prépare un composé de formule 3, où $R_1$ et $R_2$ ont les significations indiquées dans la revendication 1 et $R_3$ est un atome d'halogène, par diazotation d'un composé de formule 3 où $R_1$ et $R_2$ ont les significations indiquées dans la revendication 1 et $R_3$ est le groupe amino, avec $NaNO_2$ et HCl ou HBr, et traitement du mélange réactionnel avec du chlorure cuivreux, du bromure cuivreux ou du cuivre.

10. Procédé selon la revendication 1, caractérisé en ce qu'on convertit un composé de formule 14

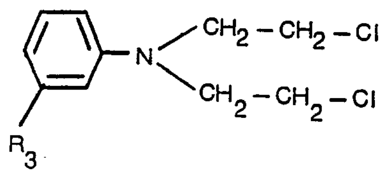

dans laquelle $R_3$ a la signification indiquée dans la revendication 1, avec un composé de formule 15

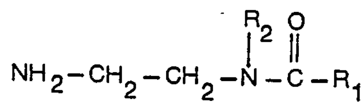

dans laquelle $R_1$ et $R_2$ ont les significations indiquées dans la revendication 1.

11. Procédé selon la revendication 1, caractérisé en ce qu'on convertit un composé de formule 16

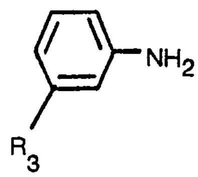

dans laquelle $R_3$ a la signification indiquée dans la revendication 1, avec un composé de formule 17

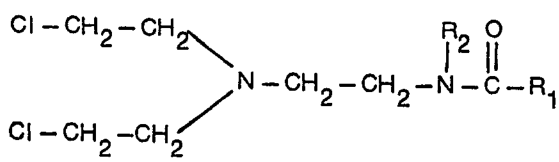

dans laquelle $R_1$ et $R_2$ ont les significations indiquées dans la revendication 1.

12. Procédé selon la revendication 1, caractérisé en ce qu'on prépare un composé de formule 3, où T est le soufre, par conversion du composé correspondant dans lequel T est l'oxygène avec du pentasulfure phosphoreux.

13. Procédé selon la revendication 1, caractérisé en ce qu'on prépare un composé de formule 3 dans lequel T est le soufre, par réaction d'un composé de formule 5 avec un composé de formule R''NCS, où R'' représente un groupe facilement éliminable.

14. Procédé selon la revendication 1, caractérisé en ce qu'on prépare un composé de formule 3 dans lequel m est 1 par oxydation du composé correspondant dans lequel m est 0.

**Patentansprüche für die Vertragsstaaten: BE CH DE FR GB IT LU NL SE**

1 Verbindungen der Formel 3

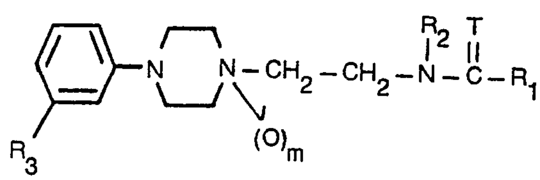

und deren Salze mit pharmazeutisch anwendbaren Säuren, in welche Formel

$R_1$ Wasserstoff, Methyl, Methoxy, Aethoxy, Amino oder Ureido ist,

$R_2$ Wasserstoff oder eine Alkylgruppe mit 1—3 Kohlenstoffatomen ist,

$R_3$ Trifluoromethyl oder Halogen ist,

T Sauerstoff oder Schwefel ist, und

m 0 oder 1 ist.

2. 1-[2-(N-Aminocarbonylamino)äthyl]-4-(3-trifluormethylphenyl)piperazin und dessen Salze mit pharmazeutisch anwendbaren Säuren.

3. Pharmazeutische Präparate, dadurch gekennzeichnet, dass sie als aktive Substanz mindestens eine Verbindung der Formel 3 enthalten, wobei $R_1$, $R_2$, $R_3$, T und m die in Anspruch 1 erwähnten Bedeutungen haben, oder ein pharmazeutisch anwendbares Säure-zusatzsalz dieser Verbindungen.

4. Ein Verfahren zur Herstellung pharmazeutisch aktiver Phenylpiperazin-Derivate, dadurch gekennzeichnet, dass antiagressiv aktive Verbindungen der Formel 3, worin $R_1$, $R_2$, $R_3$, T und m die in Anspruch 1 erwähnten Bedeutungen haben, und deren Salze mit pharmazeutisch anwendbaren Säuren in einer für die Synthese analoger Verbindungen bekannten Weise hergestellt werden.

5. Ein Verfahren nach Anspruch 5, dadurch gekennzeichnet, dass eine Verbindung der Formel 5

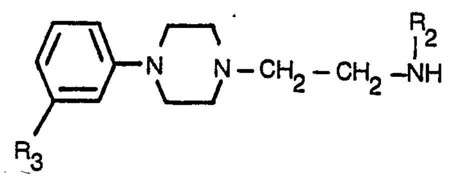

zur Reaktion gebracht wird mit

a) einem Ester oder Amid der Formylsäure oder der Acetylsäure, oder

b) ein Alkalimetallcyanat, Harnstoff oder Nitrobiuret, oder

c) ein Acetylsäurehalogenid, Acetylsäureanhydrid, das Anhydrid der Formylsäure und Acetylsäure, oder mit einem Methoxy- oder Aethoxyhalogenid, oder

d) Formylsäure oder Acetylsäure.

6. Ein Verfahren nach Anspruch 5, dadurch gekennzeichnet, dass eine Verbindung der Formel 3, in der T Schwefel ist, dadurch hergestellt wird, dass die entsprechende Verbindung in der T Sauerstoff ist mit Phosphorpentasulfid umgesetzt wird.

7. Ein Verfahren nach Anspruch 5, dadurch gekennzeichnet, dass eine Verbindung der Formel 3, in der T Schwefel ist, durch die Reaktion einer Verbindung der Formel 5 mit einer Verbindung der Formel R''NCS in der R'' für eine leicht abspaltbare Gruppe steht, hergestellt wird.

8. Ein Verfahren nach Anspruch 5, dadurch gekennzeichnet, dass eine Verbindung der Formel 3, in der m 1 ist, durch die Oxydation der entsprechenden Verbindung, in der m 0 ist, hergestellt wird.

**Patentansprüche für den Vertragsstaat: AT**

1. Ein Verfahren zur Herstellung pharmazeutisch aktiver Phenylpiperazin-Derivate, dadurch gekennzeichnet, dass antiagressiv wirksame Verbindungen der Formel 3

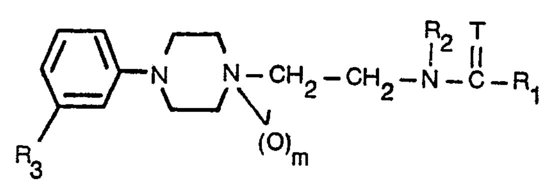

und deren Salze mit pharmazeutisch anwendbaren Säuren, in welcher Formel

$R_1$ Wasserstoff, Methyl, Methoxy, Aethoxy, Amino oder Ureido ist,

$R_2$ Wasserstoff oder eine Alkylgruppe mit 1—3 Kohlenstoffatomen ist,

$R_3$ Trifluoromethyl oder Halogen ist,

T Sauerstoff oder Schwefel ist, und

m 0 oder 1 ist.

in einer für die Synthese analoger Verbindung bekannten Weise hergestellt werden.

2. Ein Verfahren nach Anspruch 1, dadurch gekennzeichnet, dass eine Verbindung der Formel 5

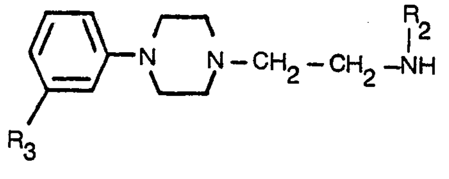

zur Reaktion gebracht wird mit

a) einem Ester oder Amid der Formylsäure oder der Acetylsäure, oder

b) ein Alkalimetallcyanat, Harnstoff oder Nitrobiuret, oder

c) ein Acetylsäurehalogenid, Acetylsäureanhydrid, das Anhydrid der Formylsäure und Acetylsäure, oder mit einem Methoxy- oder Aethoxyhalogenid, oder

d) Formylsäure oder Acetylsäure.

3. Ein Verfahren nach Anspruch 1, dadurch gekennzeichnet, dass eine Verbindung der Formel 6

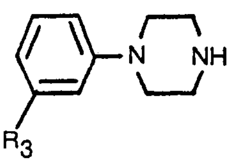

in der $R_3$ die in Anspruch 1 erwähnte Bedeutung hat, mit einer Verbindung der Formel 7

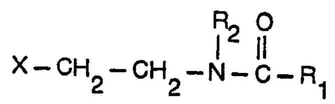

in der $R_1$ und $R_2$ die in Anspruch 1 erwähnten Bedeutungen haben, und X eine abspaltbare Gruppe ist, wie Chlor, Brom oder Tosylat, umgesetzt wird.

4. Ein Verfahren nach Anspruch 1, dadurch gekennzeichnet, dass eine Verbindung der Formel 3, in der

$R_1$ Methyl ist,

$R_2$ Alkyl mit 1 bis 3 Kohlenstoffatomen ist, und

$R_3$ die in Anspruch 1 gegebene Bedeutung hat, dadurch hergestellt wird, dass eine Verbindung der Formel 3, die nicht am Stickstoffatom substituiert ist, mit einer Verbindung $R_2Y$, in der $R_2$ die obengenannte Bedeutung hat, und Y ein Halogenatom oder eine Sulfatgruppe ist, umgesetzt wird.

5. Ein Verfahren nach Anspruch 1, dadurch gekennzeichnet, dass eine Verbindung der Formel 8

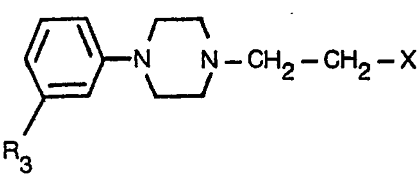

in der $R_3$ die in Anspruch 1 genannte Bedeutung hat, und X die in Anspruch 3 genannte Bedeutung hat, mit einer Verbindung der Formel 9

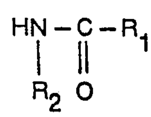

in der $R_2$ die in Anspruch 1 genannte Bedeutung hat, und $R_1$ Methyl ist, umgesetzt wird.

6. Ein Verfahren nach Anspruch 1, dadurch gekennzeichnet, dat eine Verbindung der Formel 10

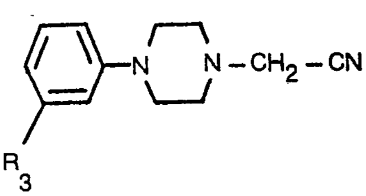

in Anwesenheit eines Katalysators in Essigsäureanhydrid, gegebenenfalls in Anwesenheit von Essigsäure, mit Wasserstoff hydriert wird.

7. Ein Verfahren nach Anspruch 1, dadurch gekennzeichnet, dass eine Verbindung der Formel 11

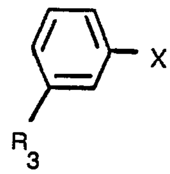

in der X die in Anspruch 3 genannte Bedeutung hat, und $R_3$ Trifluormethyl ist, mit einer Verbindung der Formel 12

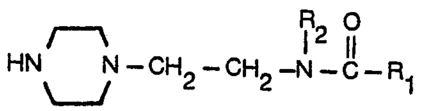

in der $R_1$ und $R_2$ die in Anspruch 1 gegebenen Bedeutungen haben, umgesetzt wird.

8. Ein Verfahren nach Anspruch 1, dadurch gekennzeichnet, dass eine Verbindung der Formel 13

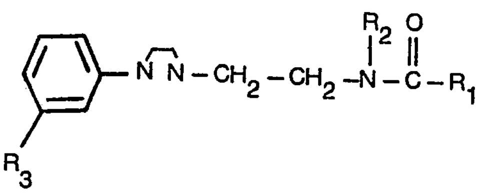

in der $R_1$, $R_2$ und $R_3$ die in Anspruch 1 gegebenen Bedeutungen haben, mit 1,2-Dibromäthan umgesetzt wird.

9. Ein Verfahren nach Anspruch 1, dadurch gekennzeichnet, dass eine Verbindung der Formel 3, in der $R_1$ und $R_2$ die in Anspruch 1 gegebenen Bedeutungen haben, und $R_3$ ein Halogenatom ist, dadurch hergestellt wird, dass eine Verbindung der Formel 3, in der $R_1$ und $R_2$ die in Anspruch 1 gegebenen Bedeutungen haben, und $R_3$ die Aminogruppe ist, mit $NaNO_2$ und HCl oder HBr diazotiert wird, und die Reaktionsmischung mit Kupfer (I)-chlorid, Kupfer (I)-bromid, der Kupfer behandelt wird.

10. Ein Verfahren nach Anspruch 1, dadurch gekennzeichnet, dass eine Verbindung der Formel 14

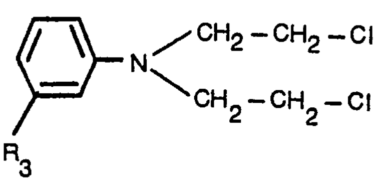

in der $R_3$ die in Anspruch 1 gegebene Bedeutung hat, mit einer Verbindung der Formel 15

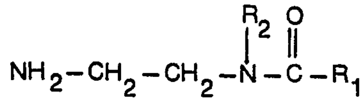

in der $R_1$ und $R_2$ die in Anspruch 1 gegebenen Bedeutungen haben, umgesetzt wird.

11. Ein Verfahren nach Anspruch 1, dadurch gekennzeichnet, dass eine Verbindung der Formel 16

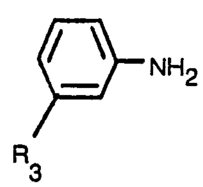

in der $R_3$ die in Anspruch 1 gegebene Bedeutung hat, mit einer Verbindung der Formel 17

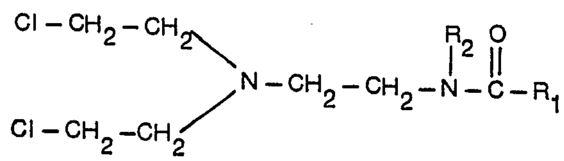

in der $R_1$ und $R_2$ die in Anspruch 1 gegebenen Bedeutungen haben, umgesetzt wird.

12. Ein Verfahren nach Anspruch 1, dadurch gekennzeichnet, dass eine Verbindung der Formel 3, in der T Schwefel ist, dadurch hergestellt wird, dass die entsprechende Verbindung in der T Sauerstoff ist, mit Phosphorpentasulfid umgesetzt wird.

13. Ein Verfahren nach Anspruch 1, dadurch gekennzeichnet, dass eine Verbindung der Formel 3, in der T Schwefel ist, durch die Reaktion einer Verbindung der Formel 5 mit einer Verbindung der Formel R''NCS, in der R'' eine leicht abspaltbare Gruppe ist, hergestellt wird.

14. Ein Verfahren nach Anspruch 1, dadurch gekennzeichnet, dass eine Verbindung der Formel 3, in der m 1 ist, durch die Oxydation der entsprechenden Verbindung in der m 0 ist, hergestellt wird.

FORMULA SHEET

1. 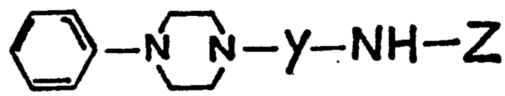

2. 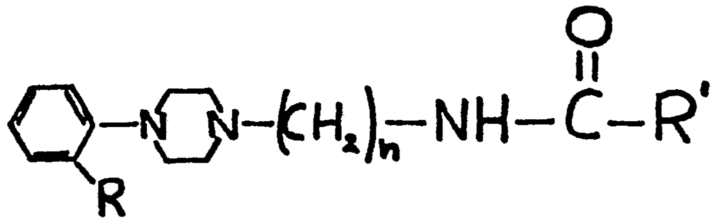

3. 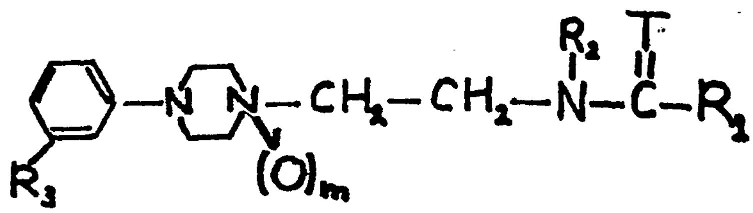

4. 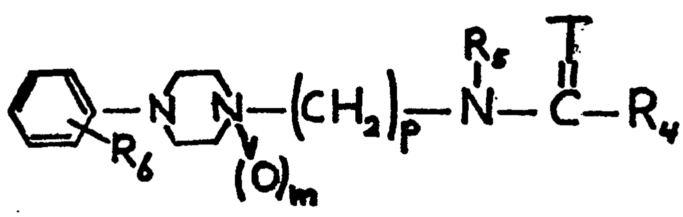

5. 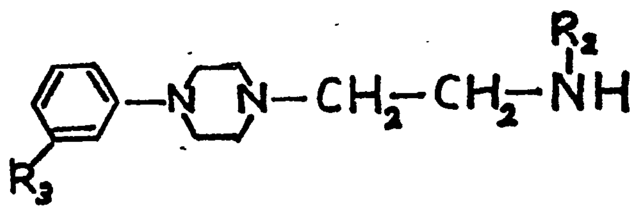

6. 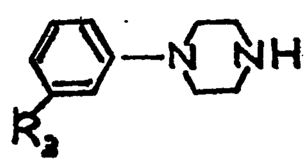

7. 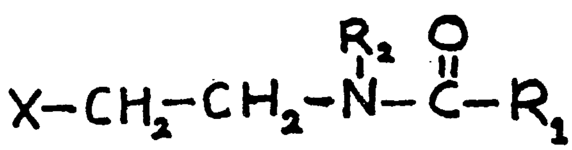

8. 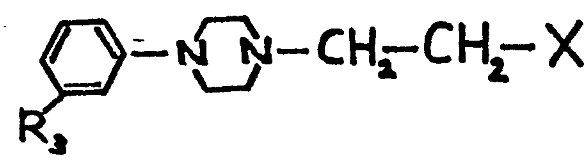

9. 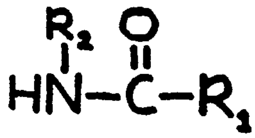

10. 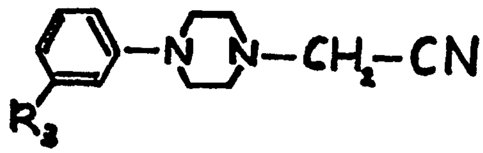

11. 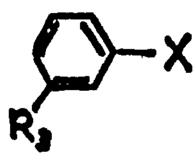

12. 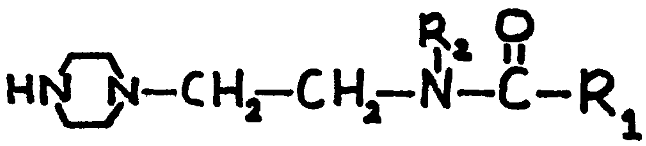

13. 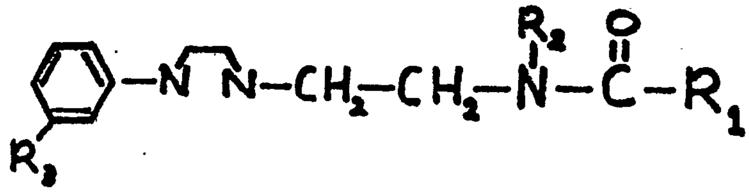

14. 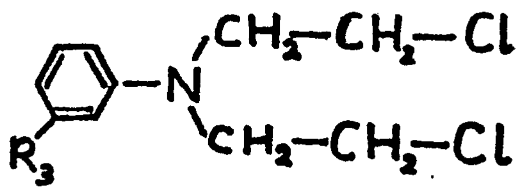

15. 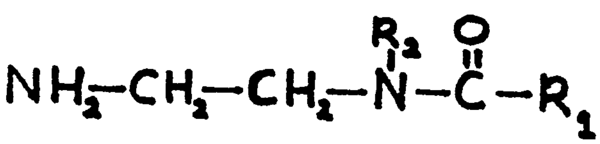

16. 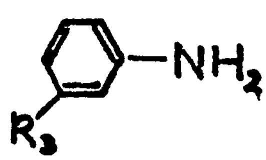

17.